# NEUE EUROPÄISCHE PATENTSCHRIFT

(11) **EP 0 809 979 B2**
(45) Veröffentlichungstag und Bekanntmachung des Hinweises auf die Entscheidung über den Einspruch: **09.04.2008**
(45) Hinweis auf die Patenterteilung: 31.07.2002
(21) Anmeldenummer: 97890084.3
(22) Anmeldetag: 06.05.1997
(51) Int. Cl.: A61C 8/00, A61F 2/28, A61B 17/68

(54) **Folie zur Abdeckung von Knochendefektstellen und Verfahren zur Herstellung der Folie**
Sheet for covering bone defect sites and process for manufacturing the sheet
Feuille pour recouvrir des emplacements de défauts osseux et procédé de fabrication de la feuille

(30) Priorität: 29.05.1996 AT 93396
(43) Veröffentlichungstag der Anmeldung: 03.12.1997
(73) Patentinhaber: MKE Metall- und Kunststoffwaren Erzeugungsgesellschaft m.b.H., 3860 Heidenreichstein (AT)
(72) Erfinder: Haas, Franz, Ing., 1010 Wien (AT)
(74) Vertreter: Müllner, Martin

(56) Entgegenhaltungen:
- EP-A- 0 621 013
- EP-A- 0 621 018
- EP-A- 0 622 052
- WO-A-92/10218
- WO-A-94/03121
- DE-C- 4 302 709
- DE-C- 4 313 192
- DE-C- 4 414 675
- DE-U- 9 413 963
- DE-U- 29 513 342
- FR-A- 2 713 090
- SU-A- 1 572 608
- US-A- 5 348 788

## Beschreibung

Die Erfindung betrifft eine Folie zur Abdeckung von Knochendefektstellen, z.B. von Defektfrakturen, Alveolen od.dgl. und zur geführten Knochenregeneration, wobei die Folie aus bleibend verformbarem Titan besteht, sowie ein Verfahren zur Herstellung der Folie.

Um Knochensubstanz bei Defekten wieder aufzubauen ist es bekannt, die Defektstelle durch eine Folie abzudecken und das Weichgewebe durch die Folie vom Hineinwachsen in die Defektstelle fernzuhalten. Es ist ferner bekannt, den Bereich, der für eine Knochenneubildung vorgesehen ist, durch ein Granulat auszufüllen und das geschaffene Volumen durch die Folie abzudecken. Das Granulat, meist Hydroxylapatit übt "Platzhalterfunktion" für den heranwachsenden Knochen aus. Folien werden meist aus textilem Material hergestellt, wobei man jene Fasern einsetzt, die sich jahrelang für das Nähen von Wunden bewährt haben. Diese Gewebe haben schützende Wirkung, wenn sie in die gewünschte Position gebracht und dort z.B. durch Vernähen lagefixiert wurden. Die Faserstruktur, die Randbereiche beim Zuschneiden, z.B. während einer Operation und die mechanischen Eigenschaften des textilen Materials, insbesondere die Elastizität, können bei vielen Anwendungsfällen nicht als ideal bezeichnet werden. In diesem Sinn ist aus der EP 621 013 A2 eine Membran aus Latex oder einem Elastomer bekannt. Diese Membran wird zwischen Knochen und Gewebe eingebracht. Die US 5 348 788 A offenbart ein dreidimensionales Netzwerk mit Vorsprüngen und sehr kleinen Poren für biomedizinische Zwecke aus Kunststoff bzw. unter Verwendung eines keramischen Materials.

Aus der EP 622 052 A1 ist ein Verfahren zum Formen einer Folie zur Förderung des Knochenwachstums im Dentalbereich bekannt. Dabei wird auf digitalem Weg ein Abbild der Defektstelle aufgenommen und ein Modell danach hergestellt, wobei eine steife Folie durch das Modell in die gewünschte Form gebracht wird. Als Folienmaterial wird formstabiles Titan in einer Stärke von 0,3 mm herangezogen. Die DE-PS 43 02 709 C1 betrifft eine Abdeckeinrichtung mit einer Abdeckmembran zum vorübergehenden Abdecken einer mit Knochenaufbaumaterial, wie Hyroxylapatitgranulat, gefüllten Defektstelle. Die Abdeckmembran ist geschmeidig und biegsam und erfordert zur Versteifung eine Abdeckmembran, die als feste, starre Titanfolie einer Stärke von 0,3 bis 1 mm mit Perforationen ausgebildet sein kann. Auch für Implantate, Nägel, Platten oder Schienen wird in der Chirurgie das Metall Titan verwendet. Die Verträglichkeit ist ausgezeichnet und Röntgenbilder werden insbesondere bei geringen Wandstärken durch Titan nicht wesentlich abgeschattet.

Die Erfindung zielt darauf ab, eine Folie zur Abdeckung von Knochendefektstellen zu verbessern, insbesondere praxisgerecht auszubilden, sodaß auch die Anwendung in kleinen Bereichen, etwa in der Kieferchirurgie möglich ist. Dies wird durch die kennzeichnenden Merkmale von Anspruch 1 erreicht. Die Titanfolie ist für die genannten Zwecke dann noch fest genug und hat keine Federwirkung, die ein Positonieren am Knochen erschwert. Die aufgerauhte oder geriffelte Oberfläche der Titanfolie stellt bei der chirurgischen Anwendung die Außenseite, also die dem Knochen abgewandte und dem Gewebe zugewandte Seite dar. Das Gewebe findet auf der Folie einen guten Halt. Ein Einwachsen der Folie wird dadurch etwa im Zahnfleischbereich begünstigt. Die Aufrauhung kann durch Sandstrahlen oder durch Prägen und die Riffelung durch Elektroerosion bzw. durch Laserbearbeitung sowie durch chemische Oberflächenbehandlung erfolgen. Eine besondere Ausführungsform der Folie ist dadurch gekennzeichnet, daß sie in Stücke von beispielsweise 3 x 4 cm zugeschnitten ist und daß ein Schnittgrat ausgebildet ist, der allseitig eine der Oberflächen überragt. Der Grat ist nach einer Richtung ausgerichtet und bildet eine ausgeprägte Dichtkante am Knochen, die ein Einwachsen des Weichgewebes verhindert. Eine Bandage, z.B. aus Nahtmaterial, wird im Anlagebereich der Folie am Knochen so angebracht, daß ein dichter Anschluß gewährleistet ist.

Eine Titanfolie wird durch Walzen in den Bereich der gewünschten Materialdicke gebracht. Der Walzvorgang bringt es mit sich, daß die Oberflächenstruktur verdichtet ist und sich daher eine Federwirkung bei einer Anpassung an den Knochen einstellt. Diese ist natürlich unerwünscht, denn es soll das Material spannungsfrei und bleibend verformbar in der gewünschten Position verharren. Um eine Titanfolie spannungsfrei und bleibend verformbar auszubilden, erfolgt nach dem Walzen eine chemische Behandlung, vorzugsweise mit Flußsäure, zum Abtragen der insbesondere beim Walzen vorgespannten Oberflächen und zur Reduzierung der Foliendicke, gegebenenfalls unter Beaufschlagung der Folie mit Ultraschall und Ausbildung einer stehenden Welle erfolgt. Durch die chemische Behandlung werden die Randschichten der Titanfolie abgetragen, also jene Schichten, die die Federwirkung begründen. Nach Erreichen der gewünschten Foliendicke von etwa 0,1 mm oder darunter erfolgt ein Waschvorgang sowie das Trocknen, z.B. im Trockenofen, bei 100°C. Eventuelle Restspannungen werden dadurch weitgehend neutralisiert.

Die spannungsfreie Titanfolie kann leicht und irreversibel in die gewünschte Form gebracht werden. Zur Lagefixierung dieser Folien für chirurgische Zwecke können Titannägel verwendet werden.

Ausführungsbeispiele des Erfindungsgegenstandes sind in den Zeichnungen dargestellt. Fig. 1 zeigt einen Querschnitt durch einen Kieferknochen mit Implantat und Abdeckfolie, Fig. 2 eine schematische Darstellung einer Defektfraktur mit Abdeckfolie und Fig. 3 den Ablauf des Verfahrens zur Herstellung einer Folie.

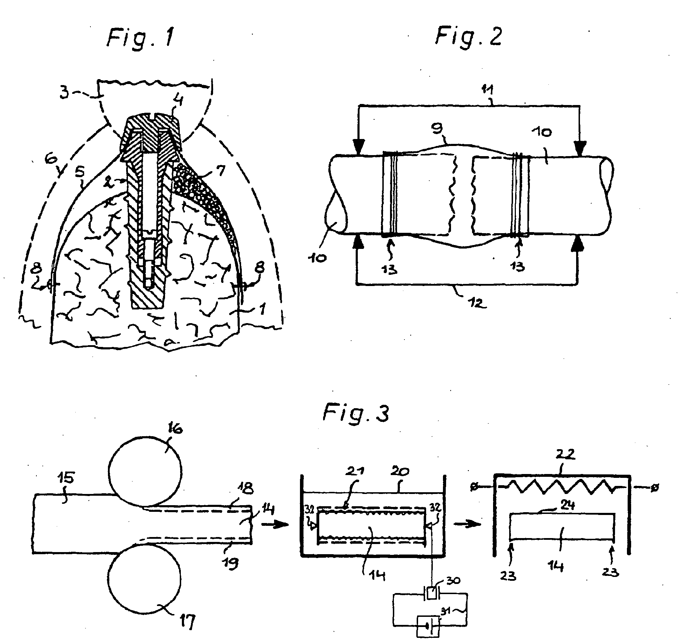

Gemäß Fig. 1 ist in einem Kieferknochen 1 ein Primärteil 2 eines Implantates eingebohrt. Die obere Öffnung, in welche nach dem Einwachsen des Primärteiles ein Sekundärteil mit einem Zahn 3 eingesetzt wird, ist durch eine Schraubkappe 4 verschlossen. Um Knochensubstanz längs des Primärteiles 2 hochzuziehen, ist eine bleibend verformbare, unelastische, spannungsfreie Titanfolie 5 einer Materialdicke von weniger als 0,1 mm, hier beispielsweise von 0,025 mm, zwischen der Schraubkappe 4 und dem Kieferknochen 1 mantelförmig angeordnet, die das Gewebe 6 vom Knochen 1 abhält und so ein Heranwachsen des Gewebes 6 an den Knochen 1 verhindert. Knochensubstanz baut sich in dem Raum zwischen der Folie 5 und dem Kieferknochen 1 auf. Das Knochenwachstum erfolgt unter der Folie 5 sehr rasch, da es durch das Gewebe 6 nicht behindert wird. Der Hohlraum unterhalb der Folie 5 kann, wie rechts in Fig. 1 dargestellt, durch Hydroxylapatit 7 ausgefüllt werden. Dieses erfüllt eine "Platzhaltefunktion" für die Knochenbildung unter der Folie 5. Das Hydroxylapatit 7 wird vom Knochen rasch durchwachsen und lagert sich vollständig in die Knochensubstanz ein.

Ein dichter Anschluß der Folie 5 ist sowohl an den Primärteil 2 des Implantates als auch an den Kieferknochen 1 erforderlich; dazu wird die Folie 5 mit einem kleinen zentralen Loch versehen und durch dieses der Schaft der Schraubkappe 4 durchgesteckt. Die Schraubkappe 4 ist konisch ausgebildet und sitzt auf einem Konus des Implantates. Die Folie 5 wird zwischen dem Kappenkonus und dem Implantatkonus eingeklemmt und sodann - unter Einbringung des Hydroxylapatits 7 an den Kieferknochen 1 mantelförmig angelegt. Die Titanfolie 5 erlaubt ein bleibendes Anformen an den Knochen 1. Durch Faltenbildung wird die Mantelform erreicht. Die Titanfolie 5 wird mit vollkommen unelastischen technologischen Eigenschaften eingesetzt, sodaß sich kein Rückfedern bei der oben beschriebenen Formgebung bzw. bei der Anpassung an das Implantat 2 und an den Kieferknochen 1 ergibt.

Die Außenfläche der Folie 5 ist rauh, damit das Gewebe 6 dort einen festen Halt findet. Der nötige Rauheitsgrad kann z.B. durch Sandstrahlen, Prägen, durch eine chemische Oberflächenbehandlung oder durch eine Laserbearbeitung, etwa durch eine Riffelung, erreicht werden. Die Folie 5 aus Titanblech verbleibt in ihrer Position und muß nicht entfernt werden. Röntgenbilder werden kaum nennenswert abgeschattet, sodaß man Veränderungen im Bereich des Implantates auch unterhalb der Folie 5 im Röntgenbild gut erkennen kann.

In Fig. 2 ist der.Einsatz einer Folie 9 im Zusammenhang mit einer Defektfraktur eines Knochens 10 dargestellt. Der Knochen 10 bzw. die Fraktur wird z.B. durch Platten oder Nägel, die symbolhaft durch Klammern mit Pfeilen 11, 12 dargestellt sind, eingerichtet. Der Frakturbereich, dem Knochenstücke fehlen, wird durch die unelastische, spannungsfreie, bleibend verformbare Folie 9 aus Titanblech manschettenartig ummantelt und im Anschlußbereich an den Knochen mit einer Bandage 13 fixiert und abgedichtet. Die Folie 9 hält das Gewebe vom Knochen 11 im Frakturbereich fern, sodaß das Wachstum des Knochens ungestört erfolgen kann. Man rechnet mit einem Knochensubstanzzuwachs von 1 mm in 10 Tagen.

Fig. 3 zeigt ein Verfahren zur Herstellung einer Folie 14 aus Titanblech 15. Durch mindestens einen walzvorgang (Walzen 16, 17) wird das Titanblech 15 auf eine Dicke von etwa 0,025 mm gebracht. Die Oberflächenbereiche 18, 19 weisen eine verdichtete Struktur auf, wodurch sich eine Federwirkung des gewalzten Materials ergibt. In einem anschließenden chemischen Bad (Säurebad) 20 wird die Oberfläche 18, 19 von zugeschnittenen Folienstücken 21 beiderseits abgetragen. Dazu ist die Anwendung von Flußsäure zweckmäßig. Durch die Anordnung im chemischen Bad (Säurebad) erreicht man die gewünschte Foliendicke von beispielsweise 0,015 bis 0,020 mm und zugleich die Spannungsfreiheit bzw. die unelastische, bleibend verformbare Materialeigenschaft. Es ist nicht das Ziel, die Folie möglichst dünn auszubilden, sondern bei maximaler Festigkeit der Folie die vorgenannten Materialeigenschaften zu verleihen.

### Beispiel 1

Eine Titanfolie, die durch Walzen auf eine Foliendicke von 0,2 mm gebracht wurde, wird in ein Flußsäurebad getaucht und verharrt dort, bis eine Foliendicke von 0,1 mm erreicht ist. Die beiderseitige Abtragung der Oberfläche von 0,05 mm erfaßt den Bereich, der infolge des Walzvorganges die hier unerwünschten elastischen Materialeigenschaften begründet. Die von 0,2 mm auf 0,1 mm Dicke durch chemische Abtragung reduzierte Folie ist unelastisch, spannungsfrei und ohne Rückfedern bleibend verformbar.

### Beispiel 2

Durch Verlängerung der Verweildauer im Flußsäurebad wurde eine Folie von ursprünglich 0,203 mm auf 0,08 mm Dicke gebraucht. Damit wurde gleichzeitig eine Rauhtiefe erreicht, die bei 0,003 bis 0,007 mm liegt und für das Anwachsen von Gewebe hervorragend geeignet ist.

### Beispiel 3

Eine gewalzte Folie mit einer Dicke von 0,2 mm wird in ein Flußsäurebad gebracht und dort mit einem.Schwingquarz eine stehende Welle im Ultraschallbereich erzeugt. Resonanz ist im elektrischen Teil der Schwingschaltung feststellbar. Die stehende Welle führt dazu, daß sich Strukturen an der Oberfläche der Titanfolie bilden, die zu einer gleichmäßigen Riffelung infolge unterschiedlicher Abtragung im Flußsäurebad führen. Eine endgültige Foliendicke zwischen 0,08 und 0,03 mm wurde erreicht.

### Beispiel 4

Durch Flußsäurebehandlung wurde eine Titanfolie von 0,08 auf eine Dicke von 0,02 mm reduziert. Diese Foliendicke eignet sich im Dentalbereich hervorragend, weil insbesondere gute Durchlässigkeit für Röntgenstrahlen gewährleistet ist.

In Fig. 3 ist ein Schwingquarz 30 in einem Stromkreis 31 dargestellt, dessen einstellbare Frequenz der Folie 14 zugeführt wird. Die Abstimmung erfolgt so, daß die Folie 14 zwischen den Einhänge- bzw. Einspannstellen 32 einer stehenden Welle folgt, sodaß sich im Flußsäurebad die gewünschte Oberflächenstruktur ergibt.

Die Folie 14 wird in der Praxis zu Folienstücken 21 rechteckig zugeschnitten, an einer Schmalseite gelocht und als Streifen in das chemische Bad gehängt. Über die Aufhängung kann der Ultraschall bzw. die Hochfrequenz des Schwingquarzes 30 angekoppelt werden, sodaß sich im Säurebad die Strukturierung des Folienstückes 21 ergibt. Nach dem Auswaschen der Säure vom Titanstreifen erfolgt der Trockenvorgang in einer Trockenkammer 22 bei beispielsweise 100°C. Die thermische Konditionierung kann letzte Reste von Spannungen neutralisieren. Vor der Wärmebehandlung erfolgt ein Sandstrahlen der Oberfläche oder ein Riffeln mittels Lasertechnik und ein Zuschneiden auf handelsübliche Maße (z.B. 3 x 4 cm), wobei das Schneiden so erfolgt, daß sich ein Grat 23 ringsum an einer Oberfläche bildet. Dieser Grat 23 liegt der rauhen Oberfläche 24 gegenüber und kommt bei der Anwendung der Folie 14 gegen den Knochen als Dichtleiste zur Wirkung.

## Patentansprüche

1. Folie zur Abdeckung von Knochendefektstellen, z.B. von Defektfrakturen, Alveolen od.dgl. und zur geführten Knochenregeneration, wobei die Folie (5, 9, 14) aus bleibend verformbarem Titan besteht, **dadurch gekennzeichnet, daß** die Folie (5,9,14) dadurch hergestellt ist, daß nach dem Walzen eine chemische Behandlung, vorzugsweise mit Flußsäure, zum Abtragen der insbesondere beim Walzen vorgespannten Oberflächen und zur Reduzierung der Foliendicke, gegebenenfalls unter Beaufschlagung der Folie mit Ultraschall und Ausbildung einer stehenden Welle, erfolgt, sodaß die Folie unelastisch spannungsfrei ist und dass die Folie eine Dicke von weniger als 0,1 mm, insbesondere weniger als 0,025 mm aufweist und mindestens eine der Oberflächen (24) aufgerauht bzw. geriffelt ausgebildet ist.

2. Folie nach Anspruch 1, **dadurch gekennzeichnet, daß** sie in Stücke von beispielsweise 3 x 4 cm zugeschnitten ist und daß ein Schnittgrat (23) ausgebildet ist, der allseitig eine der Oberflächen überragt.

3. Verfahren zur Herstellung einer Folie nach den Ansprüchen 1 oder 2, **dadurch gekennzeichnet, daß** nach dem Walzen eine chemische Behandlung, vorzugsweise mit Flußsäure, zum Abtragen der insbesondere beim Walzen vorgespannten Oberflächen und zur Reduzierung der Foliendicke, gegebenenfalls unter Beaufschlagung der Folie mit Ultraschall und Ausbildung einer stehenden Welle, erfolgt.

## Claims

1. Foil for covering bone defect sites, e.g. defective fractures, alveoli, etc., and for guided bone regeneration, wherein the foil (5, 9, 14) is made of permanently deformable titanium, **characterised in that** the foil (5, 9, 14) is manufactured in such a way that after rolling there takes place a chemical treatment, preferably with hydrofluoric acid, for removing the surfaces which have been pre-stressed in particular during rolling and for reducing the foil thickness, if necessary by subjecting the foil to ultrasound and the formation of a standing wave so that the foil is non-elastic and tension-free and that the foil has a thickness of less than 0.1 mm, in particular less than 0.025 mm, and at least one of the surfaces (24) is constructed to be rough or rippled.

2. Foil according to claim 1, **characterised in that** it is cut into pieces of approximately 3 x 4 cm and that a burr (23) is formed which projects over one of the surfaces on all sides.

3. Method for producing a foil according to claims 1 or 2, **characterised in that** after rolling there takes place a chemical treatment, preferably with hydrofluoric acid, for removing the surfaces which have been pre-stressed in particular during rolling and for reducing the foil thickness, if necessary by subjecting the foil to ultrasound and the formation of a standing wave.

## Revendications

1. Feuille pour recouvrir des zones de lésion osseuse, telles que par exemple des fractures avec lacunes, des alvéoles ou similaires et pour la régénération osseuse guidée, la feuille (5, 9, 14) se composant de titane déformable de façon durable, **caractérisée en ce que** la feuille (5, 9, 14) est fabriquée **en ce qu'**après le laminage est effectué un traitement chimique, de préférence avec de l'acide fluorhydrique, pour décaper les surfaces soumises à une précontrainte, en particulier lors du laminage, et pour réduire l'épaisseur de la feuille, le cas échéant par l'action d'ultrasons sur la feuille et formation d'une onde stationnaire, de telle sorte que la feuille est inélastique et sans contrainte et que la feuille présente une épaisseur inférieure à 0,1 mm, en particulier inférieure à 0,025 mm et présente un état rugueux ou strié sur au moins l'une des surfaces (24).

2. Feuille selon la revendication 1, **caractérisée en ce qu'**elle est découpée en pièces, par exemple de 3 x 4 cm, et **en ce qu'**elle présente une bavure de découpe (23) qui, de tous côtés, déborde de l'une des surfaces.

3. Procédé de fabrication d'une feuille selon les revendications 1 ou 2, **caractérisé en ce que**, après le laminage, est effectué un traitement chimique, de préférence avec de l'acide fluorhydrique, pour décaper les surfaces soumises à une précontrainte, en particulier pendant le laminage, et pour réduire l'épaisseur de la feuille, le cas échéant par action d'ultrasons sur la feuille et formation d'une onde stationnaire.
